(19) **Europäisches Patentamt** / **European Patent Office** / **Office européen des brevets**

(11) **EP 4 487 903 B1**

(12) **EUROPEAN PATENT SPECIFICATION**

(45) Date of publication and mention
of the grant of the patent:
**04.06.2025 Bulletin 2025/23**

(51) International Patent Classification (IPC):
***H01L 23/055*** (2006.01)   ***A61N 1/375*** (2006.01)
***H01L 23/10*** (2006.01)

(21) Application number: **23184167.7**

(22) Date of filing: **07.07.2023**

(52) Cooperative Patent Classification (CPC):
**A61N 1/3754; H01L 23/055; H01L 23/10**

(54) **HERMETIC ENCLOSURE COMPRISING THROUGH GLASS VIAS AND MEDICAL IMPLANT COMPRIS-ING SUCH A HERMETIC ENCLOSURE**

HERMETISCHE UMFASSUNG MIT GLASDURCHGÄNGEN UND MEDIZINISCHES IMPLANTAT MIT EINER SOLCHEN HERMETISCHEN UMFASSUNG

ENCEINTE HERMÉTIQUE COMPRENANT DES VIAS TRAVERSANTS EN VERRE ET IMPLANT MÉDICAL COMPRENANT UNE TELLE ENCEINTE HERMÉTIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**08.01.2025 Bulletin 2025/02**

(73) Proprietors:
• **SCHOTT AG**
**55122 Mainz (DE)**
• **Schott Primoceler Oy**
**33720 Tampere (FI)**

(72) Inventors:
• **THOMAS, Jens Ulrich**
**55122 Mainz (DE)**
• **POLOJARVI, Ville**
**33720 Tampere (FI)**
• **DAMM, Thorsten**
**55122 Mainz (DE)**
• **LAHTINEN, Ossi**
**33720 Tampere (FI)**
• **GROHMANN, Lukas**
**84028 Landshut (DE)**
• **MÄÄTTÄNEN, Antti**
**33720 Tampere (FI)**
• **LUNDÈN, Heidi**
**33720 Tampere (FI)**

(74) Representative: **Schott Corporate IP**
**Hattenbergstraße 10**
**55122 Mainz (DE)**

(56) References cited:
EP-A1- 3 812 352      US-A1- 2014 343 648
US-A1- 2021 304 973     US-B2- 7 812 416

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to a hermetic enclosure comprising at least one glass substrate having at least one electrical feedthrough configured as a through glass via which comprises a conductive rod electrically connecting the inside of the enclosure to the outside through a glass substrate.

Electronic devices can be used inside the human body for sensing (e.g., temperature, fluid composition, pressure, conductivity, chemical properties, strain) or for active treatment (e.g., cardiovascular stimulation, nerve stimulation, brain stimulation, drug delivery, drug activation). However, electronic devices should not be placed directly in the human body. Since the body environment has an average pH-value from 7.35 to 7.45 and of a temperature 36.5-37.5 °C it is potentially corrosive. Ordinary metal and plastic components are likely to degrade in such an environment, releasing toxic substances. Usually, electronic devices used as implants are sealed in housings. Titanium or titanium alloy housings are widely used for cardiac stimulation. However, they are large, heavy and bulky if they are to be made hermetic. Glass as an enclosure material could be a lightweight but robust alternative. For example, European Patent EP 3 012 059 B1 shows a method for manufacturing a transparent component for protecting an optical component. A new laser welding method is used therein.

Some electronic devices require housings having electrical feedthroughs in order to provide electrical connections to the outside. EP 3 812 352 A1 discloses a hermetic glass enclosure with vias to establish an electrical contact from the inside of the enclosure to the outside, e.g., for contacting a contact pad at the outside of the enclosure.

Such Through Glass Vias (TGV) are made of metals, mostly tungsten, that connect the inside surface of the glass substrate with the outside surface. However, when subjected to a corrosive environment the materials of the through glass vias can corrode and can even be released out of the glass.

US 2021/304973 A1 discloses a micro-electro-mechanical system (MEMS) device. In one embodiment, a cap of the device comprises an insulating material such as fused silica and the cap is provided with hermetically sealed through-glass vias (TGV). Bonding sites are formed at the positions of the TGVs, wherein the bonding sites comprise a bonding material such as gold formed on an adhesion layer such as tantalum.

It is an object of the invention to provide an improved electrical feedthrough through a glass substrate of a hermetic housing which is suitable for use in corrosive environments and in particular suitable for use as a medical implant.

Disclosure of the invention

A hermetic enclosure according to claim 1 comprising at least one glass substrate having at least one electrical feedthrough configured as a through glass via is proposed. The via comprises an electrically conductive rod electrically connecting the inside of the enclosure to the outside through the glass substrate. The part of the conductive rod's surface facing towards the outside of the enclosure is completely covered with an electrically conductive coating, wherein the electrically conductive coating is a multilayer structure comprising at least an adhesion layer in direct contact with the conductive rod and a corrosion resistant layer, wherein the electrical feedthrough comprises a depression in or an elevation on an outside facing surface of the glass substrate and/or on an inside facing surface of the glass substrate and the recess or elevation surrounds the conductive rod.

Such hermetic enclosures are formed by two or more substrates which are hermetically bonded together to hermetically enclose a function area or a cavity. For example, the hermetic enclosure may comprise three substrates. A base formed by the glass substrate comprising the at least one electrical feedthrough, a spacer substrate and a cover substrate. In this example, the base glass substrate defines a bottom wall of an enclosed cavity, the spacer substrate defines side walls of the enclosed cavity, and the cover substrate defines a top wall of the enclosed cavity. In a further example, the hermetic enclosure may comprise two substrates, a base glass substrate comprising the at least one electrical feedthrough, and a cavity substrate. The cavity substrate has a cavity produced, for example, by etching, laser assisted etching, CNC machining or laser ablation and defines side walls and a top wall of an enclosed cavity, and the base glass substrate defines a bottom wall of the enclosed cavity.

The hermetic enclosure may be directly obtained by stacking the respective substrate and subsequently bonding of the substrates. An efficient method for obtaining a large number of hermetic enclosures involves stacking and bonding of entire wafers and subsequently separating the formed enclosures, e.g., by saw dicing. In one example, a spacer wafer comprises several openings which define the cavities in conjunction with adjacent base and cover wafers. In another example, individual cavities formed in a cavity wafer define the cavities in conjunction with an adjacent base wafer.

Bonding of the substrates or wafers may, for example, be performed by means of laser bonding and/or laser welding, anodic bonding, fusion bonding, contact bonding or glass frit bonding. Bonding processes which allow direct bonding of two adjacent substrates or wafers without any intermediate material or adhesive material, such as laser bonding, are particularly preferred.

The bonding is preferably performed such that a hermetic enclosure is formed, wherein the cavity or function area is enclosed within such an enclosure. As used herein, hermetically sealed means in particular an enclosure that has a helium leakage rate of less than $1 \cdot 10^{-8}$ mbar·l/sec and is preferably in the range $1 \cdot 10^{-10}$ mbar·l/sec to $1 \cdot 10^{-9}$ mbar·l/sec.

In a preferred laser bonding process, a short-pulsed laser beam from a laser source, for which at least one of the substrates is transparent, is focused to a spot inside the formed substrate stack. By choosing the repetition rate and a scan rate of the laser, the individual laser pulses are arranged so closely together that a resulting nonlinear absorption zone of a laser pulse within the material is in contact with a neighboring nonlinear absorption zone of a further laser pulse, or even overlaps with it, such that heat accumulation can occur. Due to the accumulated heat, the material of the substrate stack is locally melted and a continuous welding "line" can be obtained. For creating such a continuous welding line, a focus plane of the laser beam is arranged close to but not at the interface between the two substrates. The laser beam is arranged at a distance below the interface between the two substrates such that the accumulated heat causes the material of the first and second substrate to locally melt and mix so that a hermetic bond is formed. The area, in which the accumulated heat of the incident laser causes the material of the two substrates to melt and to mix is designated as laser treated zone. In an area surrounding said laser treated zone, the heat introduced by the laser is insufficient to melt the material but may cause modifications of the material and/or the electrically conductive coating arranged on the glass substrate. This area is in the following referred to as heat affected zone.

Preferably, the glass substrate comprising the at least one electrical feedthrough is bonded to a further substrate, such as a spacer substrate or cavity substrate, by means of laser bonding, wherein at least one bond line is formed in which material of the glass substrate and the further substrate has been melted and mixed, wherein a distance between a laser bond line and a through glass via is preferably at least 50 $\mu$m. Additionally or alternatively, the distance between a laser bond line and a through glass via is chosen such that the through glass via is outside of the heat affected zone.

The enclosed cavity provided by the proposed hermetic enclosure is in particular suited for housing an electronic device. The device is preferably electrically connected to the electrical feedthroughs and is thus capable of sending and/or receiving of electrical signals and/or electrical current from the outside of the hermetic enclosure.

The electrical feedthroughs are configured as through glass vias and comprise conductive rods that are embedded in the glass substrate such that their front and back surfaces are accessible. Preferably, the conductive rods are made from or comprise metals like tungsten, titanium, an iron/nickel alloy, gold, silver, copper or (doped) silicon and combinations of said materials.

A length of the conductive rods is chosen such that an electrical contact may be established through the glass substrate. A thickness of the glass substrate is preferably in the range of from 200 $\mu$m to 4000 $\mu$m, more preferably from 500 $\mu$m to 1000 $\mu$m. A length of the conductive rods is preferably in the range of from 200 $\mu$m to 4000 $\mu$m, more preferably from 500 $\mu$m to 1000 $\mu$m. The lengths of the conductive rods may be chosen to be identical to the thickness of the glass substrate.

The material of the glass substrate is a glass that is preferably chosen from a borosilicate glass, such as BOROFLOAT® 33 or D263® T eco or MEMpax® available from SCHOTT AG, a quartz glass, fused silica, an alumino-borosilicate glass such as AF 32® available from SCHOTT AG, alkali-free glasses, SCHOTT B270®, or alkali-silicate glasses such as AS87.

The materials of the further substrate(s), such as the spacer substrate, the cover substrate and/or the cavity substrate, are preferably selected from a glass, a glass ceramic, a ceramic, silicon, sapphire, diamond, or other inorganic crystals. Suitable glass materials include the materials described for the glass substrate.

In case of a glass material, glasses suitable for the glass substrate are also suitable as material for the further substrate(s).

In order to protect the electrical feedthroughs from damage, for example by corrosion, the electrically conductive coating in form of a multilayer structure is provided such that the material of the conductive rod(s) of the feedthrough(s) which is exposed on the outward facing side of the enclosure and thus of the outward facing side of the glass substrate is completely covered. Thus, an outward facing side of the conductive rod is covered which includes the front surface of the conductive rod facing towards the environment and which is not surrounded by the glass substrate and is thus exposed from the glass substrate. However, the inventors have found that it is not sufficient to apply a corrosion resistant coating layer to only said front surface of the conductive rod. For example, a gold coating arranged only on said front surface of a conductive rod made from tungsten will not reduce the corrosion. Instead, it has been surprisingly found that such a gold coating will even accelerate corrosion. In order to achieve the desired corrosion resistance, not only the front surface, but the entire exposed surface of the conductive rods must be completely covered. It is believed that accelerated corrosion, for example if the enclosure is subjected to an NaCl solution in water, is caused by electrochemical processes between the material of the coating and the material of the conductive rod.

Accordingly, it is preferred to form the electrically conductive coating without any gaps such that the entire material of the conductive rod(s) which is exposed to the outside of the enclosure is covered and thus shielded from the environment. In order to achieve a gapless and complete coverage of the exposed surfaces of the metal rod(s), it is preferred to extend the electrically conductive coating to an area of the glass substrate surface adjacent to the respective conductive rod. This adjacent area preferably extends beyond the edge of the conductive rod for a distance of at least 1 $\mu$m, more preferred at least 2 $\mu$m, more preferred at least 5 $\mu$m, more preferred at least 10 $\mu$m and most preferred at least

40 $\mu$m. Since the accuracy of the patterning equipment also limits how well one can align and center the coating steps with respect to the conductive rods, it is preferred to choose the diameter of the contact pad $d_p$ greater than the diameter of the via $d_v$; so $d_p = d_v + \Delta d$, with $\Delta d > 5$ $\mu$m, more preferred 10 $\mu$m, most preferred 20 $\mu$m or more. Without loss of generality, cylindrical conduction rods and conductive contact pads are assumed. In case those cross sections are not circular, $d_v = 2r_{max}$, with $r_{max}$ the maximum radius measured from the center of the via cross section and $d_p = 2r_{min}$, with $r_{min}$ the minimum distance measured from the center of the of the contact pad cross section.

In addition to application of the electrically conductive coating to an outward facing side, it is possible to also arrange the electrically conductive coating on an inside facing side of the conductive rods. Further, it is possible to extend the electrically conductive coating over a part of a surface of the substrate in order to form electrically conductive structures. In cases where the electrically conductive coating is applied on an inside facing side of the substate and/or the conductive rods, it is preferred that said coating covers the entire inside facing side of the conductive rod, but it is also possible to cover only a part of the conductive rod's front surface with the coating.

The corrosion resistant contact layer provides both protection of the conductive rods from environmental influence and at the same time provides a reliable electrical contact surface for establishing an electrical connection. By means of this corrosion resistant contact layer, the hermetic enclosure may be used in corrosive environments including the animal and human body.

The corrosion resistant contact layer provides a contact surface which may be used to establish permanent electrical connections, for example by means of soldering a wire to the contact surface. The contact surface may also be used as part of a connector or receptacle for establishing a disconnectable electrical connection.

In case a solder connection is desired, the electrically conductive coating may be configured such that solder pads are formed.

The electrical feedthrough comprises a depression in or an elevation on an outside facing surface of the glass substrate and/or on an inside facing surface of the glass substrate, wherein the recess or elevation surrounds the electrically conductive rod, and wherein the recess or elevation is preferably flush with a front surface of the conductive rod, and wherein a depths of the depression or a height of the elevation is preferably at least 250 nm and/or less than 3 $\mu$m, preferably less than 2 $\mu$m, more preferably less than 500 nm.

Choosing the depths/height of the depression/elevation and the lengths of the conductive rod such that the conductive rod's front surface is flush with the depression/elevation, allows for a flat and homogenous surface wherein the corrosion resistant conductive coating may extend seamlessly from the front surface of the conductive rod to a part of the surface of the glass substrate. A contact pad or solder pad formed by the coating may then be chosen to have a larger surface than the size of the front surface of the conductive rod, making it easier to establish an electrical connection.

Preferably, the depths/height and/or shape of the recess or elevation are configured such that the recess or elevation serves as a flow boundary for solder. The depression or recess forms a boundary which influences and limits the flow of a solder material. If, for example, the entire area of the elevation or depression is covered with the electrically conductive coating, then the solder is only in contact with the formed solder pad and does not touch the glass substrate.

The structuring of the glass substrate provided by the recess or elevation may also serve as anchor for the coating and may thus improve adhesion of the coating.

Preferably, the electrically conductive coating is also arranged on at least a part of the glass substrate, wherein the electrically conductive coating forms a contact pad having a pad diameter $d_p$ which is larger than a via diameter $d_v$ of the conductive rod to which to contact pad is electrically connected.

The electrically conductive coating may additionally or alternatively be configured to form at least one conductive trace. Such conductive traces may form electrical connections between one or more of the electrical feedthroughs, between an electrical feedthrough and a contact pad, or between two contact pads arranged on a surface of the glass substrate. A conductive trace may also be configured to form an antenna and/or coil structure. Such a structure may be arranged on an inside facing surface and/or an outside facing surface of a substrate of the enclosure.

The electrically conductive coating is a multilayer structure having at least two layers. As the multilayer structure is electrically conductive, each of the layers is selected from an electrically conductive material.

The corrosion resistant layer is preferably configured as a diffusion barrier layer and/or a corrosion resistant contact layer. It is possible that the electrically conductive coating comprises both a diffusion barrier layer and a corrosion resistant contact layer.

Preferably, the multilayer structure of the electrically conductive coating comprises in this order the adhesion layer in direct contact with the conductive rod, at least one diffusion barrier layer, and the corrosion resistant contact layer.

The adhesive layer is chosen such that it has good adhesion on the conductive rod's material and/or on the material of the glass substrate. Suitable adhesive layers are, for example, made from or comprise Ti, Ta, Cr, Ni, NiCr, TiAl and combinations thereof.

A thickness of the adhesion layer is preferably in the range of from 2 nm to 200nm, more preferably from 10 nm to 150 nm and most preferably from 20 nm to 100 nm.

The diffusion barrier layer is chosen such that diffusion of materials from the corrosion resistant contact layer or substances from outside of the hermetic enclosure cannot diffuse or propagate towards the conductive rods and vice versa. In particular, the diffusion barrier layer is chosen such that materials contained in a solder material or an adhesive material as well as oxygen from the environment cannot damage the conductive rods of the electrical feedthroughs. Further the material of the diffusion barrier layer is preferably chosen to be biocompatible. Biocompatible materials are non-toxic and have not injurious effects on biological systems.

Preferably, the diffusion barrier layer is made from or comprises platinum (Pt), titan-nitride (TiN) and combinations thereof. If the diffusion barrier layer is the outermost layer of the multilayer structure, the material of the diffusion barrier layer is preferably chosen from a biocompatible material.

A thickness of the diffusion barrier layer is preferably in the range of from 20 nm to 200nm, more preferably from 40 nm to 150 nm and most preferably from 50 nm to 100 nm.

The corrosion resistant contact layer is preferably not only resistant to corrosive environments but is preferably also a material with good electrical conductivity and good wettability for solder materials in order to enable high quality electrical connections. Still further, as the corrosion resistant contact layer preferably forms the outermost layer of the electrically conductive coating, the corrosion resistant contact layer is preferably chosen from a biocompatible material.

Preferably, the corrosion resistant contact layer is made from or comprises gold (Au).

A thickness of the corrosion resistant contact layer is preferably in the range of from 50 nm to 200nm, more preferably from 80 nm to 150 nm and most preferably from 75 nm to 100 nm.

The electrically conductive coating may in principle be applied to the surface of the conductive rods and optionally to a part of the glass substrate's surface by means of any suitable coating method. Preferably, at least one of the layers of the electrically conductive coating is obtained by means of electroplating, electroless plating, physical vapor deposition (PVD, e.g., sputtering or evaporation, in particular resistive evaporation), electron beam deposition and/or atomic layer deposition (ALD).

Preferably, the corrosion resistant contact layer is a gold layer obtained by electroless plating. Such a process may employ the use of a seed layer to start an autocatalytic deposition of gold. Preferably, the adhesive layer or, if present, the diffusion barrier layer is chosen such that said layer serves as seed layer so that no additional seed layer is required.

Preferably, the material of the glass substrate and/or the electrically conductive coating are selected such that said materials are resistant to exposure to an NaCl solution in water, in particular to a solution of 700g/l NaCl. The enclosure may, for example, be immersed in such a solution for seven days at a temperature of 37°C and may then be examined visually for signs of corrosion. Further, it is possible to assess the corrosion resistance by determining a loss of mass. A material may then be considered to be corrosion resistant if the loss of mass is less than 5%, preferably less than 3% and most preferably less than 1%. The total mass of the electrically conductive coating is a small quantity so that a loss of mass of the material of the electrically conducting coating is hard to measure. However, if the coating is not resistant, it will develop gaps after exposure with a corrosive environment and said gaps will lead to an exposure of the conductive rods to the NaCl solution. Accordingly, the material of the electrically conductive coating is in particular considered to be corrosion resistant, if the conductive rods remain protected and thus an overall mass loss of the material of the conductive rods after exposure of the enclosure to an NaCl solution in water with 700g/l NaCl at a temperature of 37 °C for 7 days, is preferably less than 5%, more preferably less than 3% and most preferred less than 1%. Further, for the material of the glass substrate it is preferred that the mass loss of the material of the glass substrate is less than 5%, preferably less than 3% and more preferably less than 1%.

As the loss of mass of a conductive rod is a small quantity, it is preferred to use an ensemble of several enclosures to test for corrosion resistance. For example, if the enclosure has V conductive rods of mass $m_r$, an ensemble of N enclosures is used so that the total mass of the conduction rods is larger than 0.1 g:

$$M_r = N \cdot V \cdot m_r > 0.1 \text{ g.}$$

The measurement may then be performed by first drying the samples, for example in in a drying cabinet using IR-drying at 100°C for 30 min. After drying, the initial weight $M_0$ of the dry ensemble is determined, for example by means of an analytical balance (e.g., VWRI 611-3350 / LA314i from Avantor). The ensemble of enclosures is then immersed in NaCl -solution (700 g/l) for seven days at 37°C. After the testing period, the samples are rinsed with de-ionzed water and then dried, for example in a drying cabinet (IR-drying, 100°C for 30 min). After drying, the weight $M_1$ of the dry ensemble is determined, for example by means of an analytical balance (e.g., VWRI 611-3350 / LA314i from Avantor).

Hence, $\Delta M = M0 - M1$ and $\Delta M/M_r < 5\%$, $< 3\%$, most preferred less than 1%.

In order to discriminate, whether the loss in mass results from loss of mass of the conductive rods or from a corrosion of the glass (which should also be avoided), the test may be repeated on a matching ensemble using the same method with glass enclosures of the same spatial dimensions and of the same glass but with no though glass vias and thus without conductive rods. A difference in the determined mass losses $\Delta M$ between these two measurements yields the mass loss of the material of the conductive rods.

The structure of the electrically conductive coating is summarized in table 1.

(Table 1, coating properties)

| Layer | Thickness / length | function | Preferred material |
|---|---|---|---|
| Conductive rod | 200 μm to 4 mm | electrical conductor through glass substrate | Tungsten, iron/nickel alloy, Gold, Silver, Copper, Titanium, (doped) silicon |
| Adhesion layer | 2 nm to 200 nm | promotes adhesion between coating and conductive rod and/or glass substrate | Ti, Ta, Cr, Ni or alloys of those metals, such as NiCr, TiAl |
| Corrosion resistant Diffusion barrier | 20 nm to 200 nm | biocompatible diffusion barrier to prevent oxidation of conductive rod | Pt, TiN |
| Optional corrosion resistant contact layer | 50 nm to 2000 nm | inert outside facing layer with good wetting by solder | Gold |

Examples for suitable coatings applied to a glass substrate having through glass vias are given in table 2 below.

(Table 2, examples for coatings on glass substrates with through glass vias)

| | Example 1 | Example 2 | Example 3 |
|---|---|---|---|
| Glass substrate | Boro33 ©: 1500 μm | Boro33 ©: 500 μm | Fused silica: 100 μm |
| Conductive rod | Tungsten: 1500 μm | Tungsten: 500 μm | Copper: 100 μm |
| Adhesion layer | Titanium: 70 nm | Titanium: 100 nm | Nickel 50 nm |
| Corrosion resistant Diffusion barrier | Platinum: 70 nm | Platinum: 200 nm | Platinum: 50 nm |
| Optional corrosion resistant contact layer | Gold: 200 nm | - | Gold: 200 nm |

The enclosure may comprise more than one substrate made from a glass and having at least one through glass via. For example, not only the base substrate, but also the cover substrate may be configured as a glass substrate having feedthroughs protected by the electrically conductive coating having a corrosion resistant layer.

A further aspect of the invention can be seen in providing a method for producing the hermetic enclosure described herein. The method comprises providing a glass substrate having at least one electrical feedthrough configured as a through glass via and subsequent coating of a surface of the glass substrate with an electrically conductive coating. After the coating, an optional step of selectively removing the electrically conductive coating in areas between two or more through glass vias to form contact pads or to structure conductive traces may be performed.

The glass substrate is hermetically bonded to a further substrate, preferably by means of laser bonding wherein at least one bond line is formed in which material of the glass substrate and the further substrate has been melted and mixed. For laser bonding, at least one laser weld line is preferably made using an ultrashort pulse laser. Typical pulse widths are in the range of 100 fs to 100 ps. A method for carrying out such a laser bond with one or more laser welding lines is known, for example, from EP 3 012 059 B1.

The steps of coating and hermetic bonding can be performed in any order.

The hermetic enclosure described herein is in particular suitable for use as a housing for medical implants. Accordingly, a medical implant is provided comprising one of the hermetic enclosures described herein.

It is understood that the above-mentioned features and those to be explained below can be used not only in the respective combination as shown, but also in other combinations or on their own, without leaving the scope of the present invention.

Preferred embodiments of the invention are shown in the figures and will be explained in more detail in the following description, wherein identical reference numerals refer to identical or similar components or elements.

Brief description of the figures:

The figures show in schematic form:

Figure 1    a schematic cross section view from the side of a hermetic enclosure with coated through glass vias,

Figure 2    an enlarged cross-section side view of a through glass via,

Figure 3    an enclosure having a connector receptacle,

Figure 4    an enclosure comprising a clamp mechanism, and

Figure 5    an enclosure with contact pads on the opposite side of the through contacts.

Figure 1

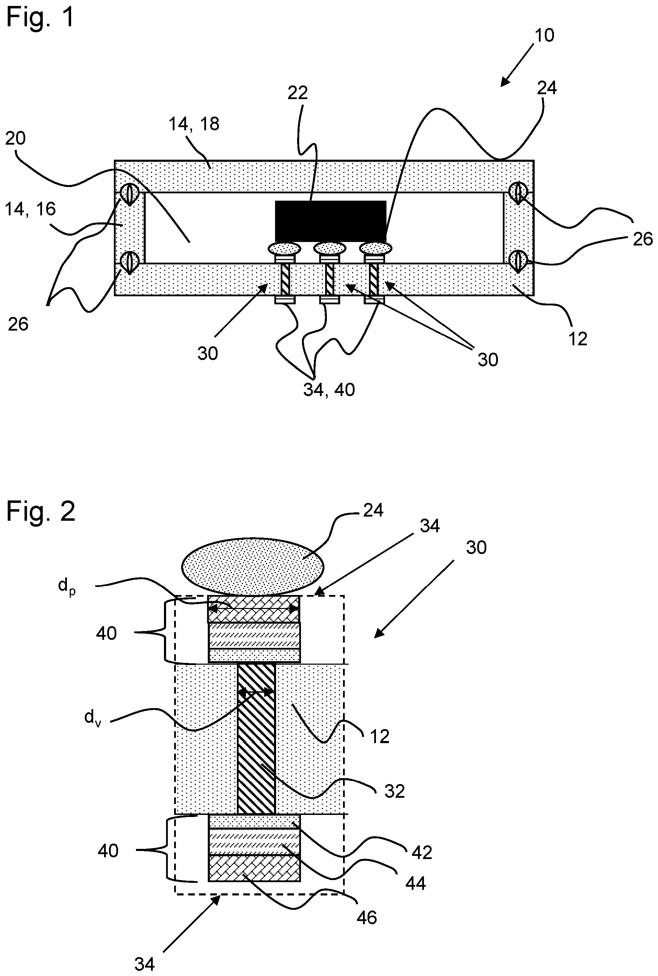

Figure 1 shows a hermetic enclosure 10 with through glass vias 30 in a schematic side view. The enclosure 10 is formed by a glass substrate 12 comprising the through glass vias 30 and further substrates 14.

In the example depicted in figure 1, the base glass substrate 12 forms the bottom of the enclosure 10. A spacer substrate 16 as a first further substrate 14 forms side walls of the enclosure 1 and a cover substrate 18 as second further substrate 14 forms a top wall of the enclosure 10. The enclosure 10 defines a cavity or function area 20.

In order to ensure that the function area 20 is hermetically enclosed, the glass substrate 12 is hermetically bonded to the spacer substrate 16 and the spacer substrate 16 is hermetically bonded to the cover substrate 18 by means of a laser bonding process. In said laser bonding process, the material at the interface of the two respective substrates 12, 16, 18 is melted and mixed in order to form bond lines 26. The weld lines 26 preferably completely surround the function area 20.

In the example shown in figure 1, an electrical device 22 is located in the function area 20 and is thus enclosed by the enclosure 10. In order to establish an electrical connection to the outside of the enclosure 10, the electrical device 22 is arranged over the through glass vias 30 and is electrically connected to said through glass vias 30 by means of a solder connection formed by solder drops 24. It is of course also possible to use other means to connect the electrical device 22 to the through glass vias 30. For example, the electrical device 22 may be located next to the through glass vias 30 on the glass substrate 12 and bond wires may be used to connect the electrical device 30 to the through glass vias 30.

For corrosion protection, an electrically conductive coating 40 configured as contact pads 34 is arranged on the outside facing side of the through glass vias 30. The detailed structure of the through glass vias 30 and the coating 40 is further described with respect to figure 2.

Figure 2 shows an enlarged cross-section side view of a through glass via 30 of the hermetic enclosure 10 shown in figure 1. The through glass via 30 comprises a metal rod as electrically conductive rod 32 which is arranged such that a front surface of the conductive rod 32 is flush with an inside facing surface of the glass substrate 12. A further front surface of the conductive rod 32 is flush with an outside facing surface of the glass substrate 12. This allows the conductive rod 32 to provide an electrically conductive connection from the inside of the enclosure 10 to the outside.

For corrosion protection of the through glass via 30 and in particular for corrosion protection of the conductive rod 32, the electrically conductive coating 40 is arranged on the outside facing front side of the conductive rod 32 and a part of the outside facing surface of the glass substrate 12.

The electrically conductive coating 40 is in the depicted embodiment configured as a layer structure having in this order an adhesion layer 42, a diffusion barrier layer 44 and a corrosion resistant contact layer 46. The coating 40 is structured to form a contact pad 34 having a diameter $d_p$ which is larger than a diameter $d_v$ of the conductive rod 32 of the through glass via 30. The material of the corrosion resistant contact layer 46 is in this example a material with good wettability for solder materials such as gold (Au).

In the layer structure of the example depicted in figure 2, the outermost layer is the corrosion resistant contact layer 46. The material of said corrosion resistant contact layer 46 is selected such that it can withstand a defined corrosive environment, such as a NaCl solution.

In an alternative embodiment, where no solder connection is required, the diffusion barrier layer can be selected from a corrosion resistant electrically conductive material and may thus serve as outermost layer.

The diffusion barrier layer is selected from a material such as platinum (Pt) which prevents diffusion of substances from the outside environment into the material of the conductive rod 32 and vice versa. In particular, the diffusion barrier layer is chosen such that it prevents diffusion of oxygen into the material of the conductive rods 32.

In this embodiment, the electrically conductive coating 40 is both arranged on the outside facing side as well as on the inside facing side of the glass substrate 12.

The electrically conductive coating 40 on the inside facing side serves as a contact pad 34 for the solder drop 24 which electrically connects the electrical device 22, see figure 1, to the through glass via 30.

In further embodiments, a part of the surface of the glass substrate 12 surrounding the conductive rod 32 could be raised or lowered compared to the remaining surface of the glass substrate 12 to form an elevation or depression surrounding the conductive rod 32. The raised or lowered area is preferably flush with the end surface of the conductive rod 32. Preferably, the electrically conductive coating 40 forming the contact pad 34 covers the entire area of said elevation or depression. Such an elevation or depression may be applied to control the flow of a solder material, wherein preferably said solder material would be confined to the elevation or depression.

The corrosion-resistant properties of the electrically conductive coating 40 are not required on the inside facing surface as the enclosure 10 protects the enclosed function area 20 from any corrosive influence from the outside. However, having the same electrically conductive coating 40 on both surfaces allows the use of the same coating process and results in a symmetrical glass substrate 12 so that any of the two sides may face towards the spacer substrate 16.

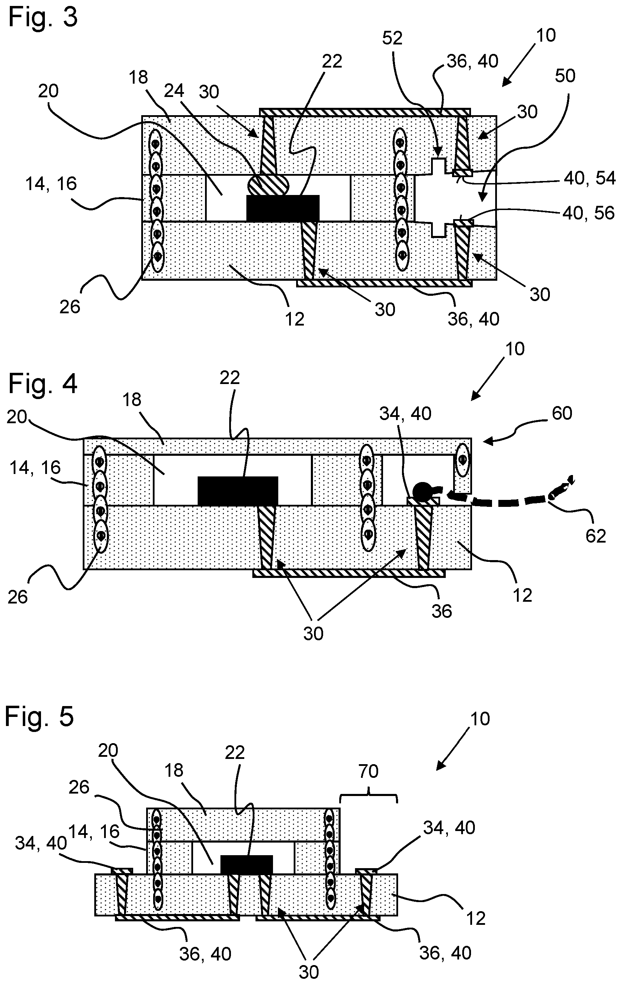

Fig. 3 shows a cross-section view of an enclosure 10 formed by the glass substrate 12, the spacer substrate 16 and the cover substate 18. The three substrates 12, 16, 18 are bonded together via bond lines 26. The enclosure 1 hermetically encloses the function area 20 which receives the electrical device 22. Further the three substrates 12, 16 and 18 define a connector receptacle area 50 which allows the insertion of a suitable connector.

Said connector receptacle area 50 is in electrical contact with the electrical device 22 by means of through glass vias 30 and conductive traces 36. The traces 36 are defined by structuring the electrically conductive coating 40 arranged on the respective outside facing surfaces of the glass substrate 12 and the cover substrate 18.

As it is not intended to form a solder connection to the conductive traces 36, the electrically conductive coating 40 arranged on the outside facing surfaces of the glass substrate 12 and the cover substrate 16 may be configured as a two-layer structure comprising the adhesion layer (42) and the barrier layer (44) as corrosion resistant layer.

Within the connector receptacle area 50, an upper contact 54 and a lower contact 56 are defined by structuring of the electrically conductive coating 40 arranged on the inside facing surfaces of the glass substrate 12 and the cover substrate 18. Further, for mechanically securing of a connector, connector notches 52 are formed in the glass substrate 12 and the cover substrate 18. The connector notches 52 are configured to receive latching elements of the connector.

Figure 4 shows another enclosure 10, similar to the enclosure described with respect to figure 3. In contrast to the embodiment of figure 3, the substrates 12, 16, 18 are configured to form a clamp 60 designed to receive and hold a nerve 62.

Within the clamp 60, the electrically conductive coating 40 is structured to form a contact pad 40 for electrically contacting the clamped nerve 62. This allows, for example, the nerve 62 to be in electrical contact with the electrical device 22 and to stimulate the nerve 62 by electrical pulses from the electrical device 22.

Figure 5 shows another enclosure 10, where electrical contacts 34 are provided on the upper surface of the glass substrate 12 on either side of the enclosed function area 20. The two electrical contact pads 34 are obtained by structuring the electrically conductive coating 40. The respective electrical contact pads 34 are connected to the back side of the glass substrate 12 by through glass vias 30 and the electrical devices 22 is likewise connected to through glass vias 30. Conductive traces 36 formed on the outside facing surface of the glass substrate 12 by structuring of the electrically conductive coating 40 establish an electrical connection between two of the through glass vias 30. The electrically conductive coating 40 is structured such that the exposed surfaces of the conductive rods 32 of the through glass vias 30, see figure 2, are covered by the coating 40.

The arrangement shown in figure 5 allows the spacer substrate 16 as well as the cover substrate 18 to remain free from electrical contacts and through glass vias 30 while still providing access to the contact pads 34 from an upper side of the enclosure 1 via a free space 70 located above the contact pads 34. Thus, optical properties of the spacer substrate 16 and the cover substrate 18 are not impaired.

Although the present invention has been described with reference to preferred examples of embodiments, it is not limited thereto but can be modified in a variety of ways, as long as the modifications fall within the scope of the appended claims.

List of reference numerals

10     enclosure
12     glass substrate
14     further substrate
16     spacer substrate
18     cover substrate
20     function area
22     electrical device
24     solder drop
26     bond line
30     through glass via
32     conductive rod

| 34 | contact pad |
|---|---|
| 36 | trace |
| 40 | conductive coating |
| 42 | adhesion layer |
| 44 | diffusion barrier layer |
| 46 | contact layer |
| 50 | connector receptacle area |
| 52 | connector notch |
| 54 | upper contact |
| 56 | lower contact |
| 60 | clamp |
| 62 | nerve |
| 70 | free space |
| $d_v$ | diameter through glass via |
| $d_p$ | diameter contact pad |

**Claims**

1. Hermetic enclosure (10) comprising at least one glass substrate (12) having at least one electrical feedthrough configured as a through glass via (30), the through glass via (30) comprising a conductive rod (32) electrically connecting the inside of the hermetic enclosure (10) to the outside through the glass substrate (12), wherein the part of the conductive rod's (32) surface facing towards the outside of the hermetic enclosure (10) is completely covered with an electrically conductive coating (40), wherein the electrically conductive coating (40) is a multilayer structure comprising at least an adhesion layer (42) in direct contact with the conductive rod (32) and a corrosion resistant layer, **characterized in that** the electrical feedthrough comprises a depression in or an elevation on an outside facing surface of the glass substrate (12) and/or on an inside facing surface of the glass substrate (12), wherein the recess or elevation surrounds the conductive rod (32).

2. Hermetic enclosure (10) according to claim 1, wherein the recess or elevation is flush with a front surface of the conductive rod (32), and/or wherein a depths of the depression or a height of the elevation is at least 250 nm and/or less than 3 μm.

3. Hermetic enclosure (10) according to claim 1 or 2, wherein the depths/height and/or shape of the recess or elevation are configured such that the recess or elevation serves as a flow boundary for solder, preferably such that the solder does not touch the glass substrate (12).

4. Hermetic enclosure (10) according to any one of claims 1 to 3, wherein the corrosion resistant layer is configured as diffusion barrier layer (44) and/or a corrosion resistant contact layer (46), wherein the multilayer structure of the electrically conductive coating (40) comprises in this order the adhesion layer (42) in direct contact with the conductive rod (32), at least one diffusion barrier layer(44) and a corrosion resistant contact layer (46).

5. Hermetic enclosure (10) according to claim 4, wherein the adhesive layer (42) is made from or comprises Ti, Ta, Cr, Ni, NiCr, TiAl and combinations thereof.

6. Hermetic enclosure (10) according to claim 4 or 5, wherein the diffusion barrier layer (44) is made from or comprises platinum (Pt), titan-nitride (TiN) and combinations thereof.

7. Hermetic enclosure (10) according to any one of claims 1 to 6, wherein the corrosion resistant contact layer (46) is made from or comprises gold (Au).

8. Hermetic enclosure (10) according to claim 7, wherein the corrosion resistant contact layer (46) is a gold layer obtained by electroless plating.

9. Hermetic enclosure (10) according to any one of claims 1 to 8, wherein the conductive rods (32) are made from or comprise tungsten titanium, an iron/nickel alloy, gold, silver, copper, silicon, and combinations of said materials.

10. Hermetic enclosure (10) according to any one of claims 1 to 9, wherein the electrically conductive coating (40) is also arranged on at least a part of a surface the glass substrate (12), wherein the electrically conductive coating (40) forms

a contact pad (34) having a pad diameter $d_p$ which is larger than a via diameter $d_v$ of the conductive rod (32) to which to contact pad (34) is electrically connected, and/or
wherein the electrically conductive coating (40) forms at least one conductive trace (36) which preferably electrically connects two or more through glass vias (30).

11. Hermetic enclosure (10) according to any one of claims 1 to 10, wherein at least one of the layers of the electrically conductive coating (40) is obtained by means of electroplating, electroless plating, physical vapor deposition (PVD), electron beam deposition and/or atomic layer deposition (ALD).

12. Hermetic enclosure (10) according to any one of claims 1 to 11, wherein the glass substrate (12) is bonded to a further substrate (14) by means of laser bonding and/or laser welding, wherein at least one bond line (26) is formed in which material of the glass substrate (12) and the further substrate (14) has been melted and mixed, wherein a distance between a laser bond line (26) and a through glass via (30) is at least 50 $\mu$m.

13. Hermetic enclosure (10) according to any one of claims 1 to 12, wherein the material of the glass substrate (12) is selected from a borosilicate glass, a quart glass, fused silica, an alumino-borosilicate glass, alkali-free glasses or alkali-silicate glasses.

14. Method for producing a hermetic enclosure (10) according to any one of claims 1 to 13, comprising the steps of

- providing a glass substrate (12) having at least one electrical feedthrough configured as a through glass via (30),
- coating of a surface of the glass substrate (12) with an electrically conductive coating (40) and optionally subsequently selectively removing the electrically conductive coating (40) in areas between two or more through glass vias (30) to form contact pads (40) and/or conductive traces (36),
- hermetic bonding of the glass substrate (12) to a further substrate (14) by means of laser bonding wherein at least one bond line (26) is formed in which material of the glass substrate (12) and the further substrate (14) has been melted and mixed,

wherein the steps of coating and hermetic bonding can be performed in any order.

15. Medical implant comprising a hermetic enclosure (10) according to any one of claims 1 to 13 or obtained by the method of claim 14.

**Patentansprüche**

1. Hermetische Umschließung (10), umfassend mindestens ein Glassubstrat (12), das mindestens eine elektrische Durchführung aufweist, die als ein Durchglas-Durchkontaktierung, Through Glass Via, (30) ausgestaltet ist, wobei der Through Glass Via (30) einen leitfähigen Stab (32) umfasst, der das Innere der hermetischen Umschließung (10) elektrisch durch das Glassubstrat (12) hindurch nach außen verbindet, wobei der Teil der Oberfläche des leitfähigen Stabs (32), der zur Außenseite der hermetischen Umschließung (10) weist, vollständig mit einer elektrisch leitfähigen Beschichtung (40) bedeckt ist, wobei die elektrisch leitfähige Beschichtung (40) eine Mehrschichtstruktur ist, die mindestens eine Haftschicht (42) in direktem Kontakt mit dem leitfähigen Stab (32) und eine korrosionsbeständige Schicht umfasst, **dadurch gekennzeichnet, dass** die elektrische Durchführung eine Vertiefung in oder eine Erhebung auf einer nach außen weisenden Oberfläche des Glassubstrats (12) und/oder auf einer nach innen weisenden Oberfläche des Glassubstrats (12) umfasst, wobei die Vertiefung oder Erhebung den leitfähigen Stab (32) umgibt.

2. Hermetische Umschließung (10) nach Anspruch 1, wobei die Vertiefung oder Erhebung bündig mit einer vorderen Oberfläche des leitfähigen Stabs (32) ist und/oder wobei eine Tiefe der Vertiefung oder eine Höhe der Erhebung mindestens 250 nm und/oder weniger als 3 $\mu$m beträgt.

3. Hermetische Umschließung (10) nach Anspruch 1 oder 2, wobei die Tiefen/Höhe und/oder Form der Vertiefung oder Erhebung so ausgestaltet ist/sind, dass die Vertiefung oder Erhebung als Flussgrenze für Lot dient, vorzugsweise so, dass das Lot das Glassubstrat (12) nicht berührt.

4. Hermetische Umschließung (10) nach einem der Ansprüche 1 bis 3, wobei die korrosionsbeständige Schicht als Diffusionsbarriereschicht (44) und/oder eine korrosionsbeständige Kontaktschicht (46) ausgestaltet ist, wobei die Mehrschichtstruktur der elektrisch leitfähigen Beschichtung (40) in dieser Reihenfolge die Haftschicht (42) in direktem Kontakt mit dem leitfähigen Stab (32), mindestens eine Diffusionsbarriereschicht (44) und eine korrosionsbeständige Kontaktschicht (46) umfasst.

5. Hermetische Umschließung (10) nach Anspruch 4, wobei die Haftschicht (42) aus Ti, Ta, Cr, Ni, NiCr, TiAl und Kombinationen davon hergestellt ist oder diese umfasst.

6. Hermetische Umschließung (10) nach Anspruch 4 oder 5, wobei die Diffusionsbarriereschicht (44) aus Platin (Pt), Titannitrid (TiN) und Kombinationen davon hergestellt ist oder diese umfasst.

7. Hermetische Umschließung (10) nach einem der Ansprüche 1 bis 6, wobei die korrosionsbeständige Kontaktschicht (46) aus Gold (Au) hergestellt ist oder dieses umfasst.

8. Hermetische Umschließung (10) nach Anspruch 7, wobei die korrosionsbeständige Kontaktschicht (46) eine Goldschicht ist, die durch stromloses Plattieren erhalten wird.

9. Hermetische Umschließung (10) nach einem der Ansprüche 1 bis 8, wobei die leitfähigen Stäbe (32) aus Wolfram-Titan, einer Eisen/Nickel-Legierung, Gold, Silber, Kupfer, Silicium und Kombinationen dieser Materialien hergestellt sind oder diese umfassen.

10. Hermetische Umschließung (10) nach einem der Ansprüche 1 bis 9, wobei die elektrisch leitfähige Beschichtung (40) auch auf mindestens einem Teil einer Oberfläche des Glassubstrats (12) angeordnet ist, wobei die elektrisch leitfähige Beschichtung (40) ein Kontaktpad (34) mit einem Paddurchmesser $d_p$ bildet, der größer als ein Via-Durchmesser $d_v$ des leitfähigen Stabs (32) ist, mit dem das Kontaktpad (34) elektrisch verbunden ist, und/oder wobei die elektrisch leitfähige Beschichtung (40) mindestens eine Leiterbahn (36) bildet, die vorzugsweise zwei oder mehr Through Glass Vias (30) elektrisch verbindet.

11. Hermetische Umschließung (10) nach einem der Ansprüche 1 bis 10, wobei mindestens eine der Schichten der elektrisch leitfähigen Beschichtung (40) durch Galvanisieren, stromloses Plattieren, physikalische Gasphasenabscheidung (PVD), Elektronenstrahlabscheidung und/oder Atomschichtabscheidung (ALD) erhalten wird.

12. Hermetische Umschließung (10) nach einem der Ansprüche 1 bis 11, wobei das Glassubstrat (12) mittels Laserbonden und/oder Laserschweißen an ein weiteres Substrat (14) gebondet ist, wobei mindestens eine Bondlinie (26) gebildet ist, in der Material des Glassubstrats (12) und des weiteren Substrats (14) geschmolzen und gemischt wurde, wobei ein Abstand zwischen einer Laserbondlinie (26) und einem Through Glass Via (30) mindestens 50 μm beträgt.

13. Hermetische Umschließung (10) nach einem der Ansprüche 1 bis 12, wobei das Material des Glassubstrats (12) ausgewählt ist aus einem Borosilikatglas, einem Quarzglas, Kieselglas, einem Alumino-Borosilikatglas, alkalifreien Gläsern oder Alkalisilikatgläsern.

14. Verfahren zum Herstellen einer hermetischen Umschließung (10) nach einem der Ansprüche 1 bis 13, umfassend die Schritte von

- Bereitstellen eines Glassubstrats (12), das mindestens eine elektrische Durchführung aufweist, die als ein Through Glass Via (30) ausgestaltet ist,
- Beschichten einer Oberfläche des Glassubstrats (12) mit einer elektrisch leitfähigen Beschichtung (40) und gegebenenfalls anschließendes selektives Entfernen der elektrisch leitfähigen Beschichtung (40) in Bereichen zwischen zwei oder mehr Through Glass Vias (30), um Kontaktpads (40) und/oder Leiterbahnen (36) zu bilden,
- hermetisches Bonden des Glassubstrats (12) an ein weiteres Substrat (14) mittels Laserbonden, wobei mindestens eine Bondlinie (26) gebildet wird, in der Material des Glassubstrats (12) und des weiteren Substrats (14) geschmolzen und gemischt wurde,

wobei die Schritte des Beschichtens und des hermetischen Bondens in beliebiger Reihenfolge durchgeführt werden können.

15. Medizinisches Implantat, das eine hermetische Umschließung (10) nach einem der Ansprüche 1 bis 13 umfasst oder durch das Verfahren nach Anspruch 14 erhalten wird.

**EP 4 487 903 B1**

**Revendications**

1. Enceinte hermétique (10) comprenant au moins un substrat en verre (12) présentant au moins une traversée électrique configurée en tant que trou d'interconnexion en verre (30), le trou d'interconnexion en verre (30) comprenant une tige conductrice (32) connectant électriquement l'intérieur de l'enceinte hermétique (10) à l'extérieur par le biais du substrat en verre (12), dans lequel la partie de la surface de la tige conductrice (32) orientée vers l'extérieur de l'enceinte hermétique (10) est entièrement recouverte d'un revêtement électroconducteur (40), dans laquelle le revêtement électroconducteur (40) est une structure multicouche comprenant au moins une couche d'adhésion (42) en contact direct avec la tige conductrice (32) et une couche résistante à la corrosion, **caractérisée en ce que** la traversée électrique comprend un creux dans ou une élévation sur une surface orientée vers l'extérieur du substrat en verre (12) et/ou sur une surface orientée vers l'intérieur du substrat en verre (12), dans lequel le creux ou l'élévation entoure la tige conductrice (32).

2. Enceinte hermétique (10) selon la revendication 1, dans laquelle le creux ou l'élévation affleure une surface avant de la tige conductrice (32), et/ou dans laquelle une profondeur du creux ou une hauteur de l'élévation est d'au moins 250 nm et/ou inférieure à 3 $\mu$m.

3. Enceinte hermétique (10) selon la revendication 1 ou 2, dans laquelle la profondeur/hauteur et/ou la forme du creux ou de l'élévation sont configurées de telle sorte que le creux ou l'élévation serve de limite d'écoulement pour une soudure, de préférence de telle sorte que la soudure ne touche pas le substrat en verre (12).

4. Enceinte hermétique (10) selon l'une quelconque des revendications 1 à 3, dans laquelle la couche résistante à la corrosion est configurée en tant que couche barrière de diffusion (44) et/ou une couche de contact résistante à la corrosion (46), dans laquelle la structure multicouche du revêtement électroconducteur (40) comprend dans cet ordre la couche d'adhésion (42) en contact direct avec la tige conductrice (32), au moins une couche barrière de diffusion (44) et une couche de contact résistante à la corrosion (46).

5. Enceinte hermétique (10) selon la revendication 4, dans laquelle la couche adhésive (42) est réalisée en, ou comprend du Ti, Ta, Cr, Ni, NiCr, TiAl et des combinaisons de ceux-ci.

6. Enceinte hermétique (10) selon la revendication 4 ou 5, dans laquelle la couche barrière de diffusion (44) est réalisée en ou comprend du platine (Pt), du nitrure de titane (TiN) et des combinaisons de ceux-ci.

7. Enceinte hermétique (10) selon l'une quelconque des revendications 1 à 6, dans laquelle la couche de contact résistante à la corrosion (46) est réalisée en ou comprend de l'or (Au).

8. Enceinte hermétique (10) selon la revendication 7, dans laquelle la couche de contact résistante à la corrosion (46) est une couche d'or obtenue par dépôt autocatalytique.

9. Enceinte hermétique (10) selon l'une quelconque des revendications 1 à 8, dans laquelle les tiges conductrices (32) sont réalisées en ou comprennent du tungstène-titane, un alliage fer/nickel, de l'or, de l'argent, du cuivre, du silicium, et des combinaisons desdits matériaux.

10. Enceinte hermétique (10) selon l'une quelconque des revendications 1 à 9, dans laquelle le revêtement électroconducteur (40) est également agencé sur au moins une partie d'une surface du substrat en verre (12), dans laquelle le revêtement électroconducteur (40) forme un plot de contact (34) ayant un diamètre de plot $d_p$ qui est plus grand qu'un diamètre de trou d'interconnexion $d_v$ de la tige conductrice (32) à laquelle le plot de contact (34) est connecté électriquement, et/ou dans lequel le revêtement électroconducteur (40) forme au moins un tracé conducteur (36) qui, de préférence, connecte électriquement deux trous d'interconnexion en verre (30) ou plus.

11. Enceinte hermétique (10) selon l'une quelconque des revendications 1 à 10, dans laquelle au moins une des couches du revêtement électroconducteur (40) est obtenue par des moyens de dépôt électrolytique, dépôt autocatalytique, dépôt physique en phase vapeur (PVD), dépôt par faisceau d'électrons et/ou dépôt de couche atomique (ALD).

12. Enceinte hermétique (10) selon l'une quelconque des revendications 1 à 11, dans laquelle le substrat en verre (12) est lié à un autre substrat (14) au moyen d'une liaison au laser et/ou d'un soudage au laser, dans laquelle au moins une ligne de liaison (26) est formée dans laquelle le matériau du substrat en verre (12) et de l'autre substrat (14) a été fondu et mélangé, dans laquelle une distance entre une ligne de liaison au laser (26) et un trou d'interconnexion en verre (30) est d'au moins 50 μm.

13. Enceinte hermétique (10) selon l'une quelconque des revendications 1 à 12, dans laquelle le matériau du substrat en verre (12) est choisi parmi un verre borosilicaté, un verre de quartz, une silice fondue, un verre alumino-borosilicaté, des verres sans alcali ou des verres alcali-silicatés.

14. Procédé de fabrication d'une enceinte hermétique (10) selon l'une quelconque des revendications 1 à 13, comprenant les étapes de

- fourniture d'un substrat en verre (12) présentant au moins une traversée électrique configurée en tant que trou d'interconnexion en verre (30),
- revêtement d'une surface du substrat en verre (12) avec un revêtement électroconducteur (40) et éventuellement enlèvement sélectif ultérieur du revêtement électroconducteur (40) dans des zones entre deux trous d'interconnexion en verre (30) ou plus pour former des plots de contact (40) et/ou des tracés conducteurs (36),
- liaison hermétique du substrat en verre (12) à un autre substrat (14) au moyen d'une liaison au laser dans laquelle au moins une ligne de liaison (26) est formée dans laquelle les matériaux du substrat en verre (12) et de l'autre substrat (14) ont été fondus et mélangés,

dans lequel les étapes de revêtement et de liaison hermétique peuvent être effectuées dans un ordre quelconque.

15. Implant médical comprenant une enceinte hermétique (10) selon l'une quelconque des revendications 1 à 13 ou obtenu par le procédé selon la revendication 14.

Fig. 1

Fig. 2

## Fig. 3

## Fig. 4

## Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3012059 B1
- EP 3812352 A1

- US 2021304973 A1